# EUROPEAN PATENT SPECIFICATION

(11) **EP 2 419 391 B1**
(45) Date of publication and mention of the grant of the patent: **30.04.2014**
(21) Application number: 10764497.3
(22) Date of filing: 08.04.2010
(51) Int. Cl.: C07C 13/62, C07D 213/38, C09K 11/06, H01L 51/50

(54) **NOVEL FUSED POLYCYCLIC COMPOUND AND ORGANIC LIGHT EMITTING DEVICE**
NEUE KONDENSIERTE POLYZYKLISCHE VERBINDUNG UND ORGANISCHE LICHTEMITTIERENDE VORRICHTUNG DAMIT
NOUVEAU COMPOSÉ POLYCYCLIQUE CONDENSÉ ET DISPOSITIF ÉLECTROLUMINESCENT ORGANIQUE

(30) Priority: 16.04.2009 JP 2009099943
(43) Date of publication of application: 22.02.2012
(73) Proprietor: Canon Kabushiki Kaisha, Tokyo 146-8501 (JP)
(72) Inventor: YAMADA, Naoki, Tokyo 146-8501 (JP); KAMATANI, Jun, Tokyo 146-8501 (JP); HORIUCHI, Takayuki, Tokyo 146-8501 (JP); TOMONO, Hiroyuki, Tokyo 146-8501 (JP); SAITOH, Akihito, Tokyo 146-8501 (JP)
(74) Representative: TBK
(86) International application number: PCT/JP2010/056727
(87) International publication number: WO 2010/119914

(56) References cited:
- WO-A1-2008/120806
- WO-A1-2008/120808
- WO-A1-2008/120808
- WO-A1-2008/146720
- JP-A- 2003 238 516
- JP-A- 2005 089 674
- WANG, K. K. ET AL.: 'Thermolysis of Benzoenyne-Allenes To Form Biradicals and Subsequent Intramolecular Trapping with a Tetraarylallene To Generate Two Triarylmethyl Radical Centers' THE JOURNAL OF ORGANIC CHEMISTRY vol. 64, no. 5, 1999, pages 1650 - 1656

## Description

### TECHNICAL FIELD

The present invention relates to a novel fused polycyclic compound and an organic light emitting device using the compound.

### BACKGROUND ART

Recent progress of an organic light emitting device is remarkable.

However, the present situation requires an optical output with additionally high luminance or high conversion efficiency. In addition, many problems still remain to be solved regarding durability against, for example, a change over time due to long-term use or deterioration due to oxygen, moisture, or the like.

Further, when an application to a full-color display or the like is intended, a device must emit blue light having a good color purity with high efficiency. However, those problems have also not been sufficiently solved yet. Meanwhile, an organic light emitting device having a particularly high color purity, particularly high light emitting efficiency, and particularly high durability, and a material for realizing the device have been requested.

Attempts have been made to use organic compounds each having a benzofluoranthene skeleton in light emitting devices to solve the above-mentioned problems (Japanese Patent Application Laid-Open No. H10-189247 and Japanese Patent Application Laid-Open No. 2005-235787).

However, the devices must be further improved from the viewpoints of emission hue, efficiency, luminance, and durability.

Meanwhile, synthesis examples of organic compounds each having a diindenochrysene skeleton have been reported (J. Org. Chem., 64, 1650-1656 (1999)).

The organic compounds described in the above-mentioned patent documents and organic light emitting devices having the compounds are susceptible to improvement from the viewpoint of commercialization.

To be specific, an optical output with additionally high luminance or high conversion efficiency is needed for the commercialization. In addition, an improvement in durability against, for example, a change over time due to long-term use or deterioration due to oxygen, moisture, or the like is needed.

Further, an organic light emitting device requested when an application to a full-color display or the like is intended must emit blue light having a good color purity with high efficiency. However, those problems have also not been sufficiently solved yet.

Therefore, an organic light emitting device having a particularly high color purity, particularly high light emitting efficiency, and particularly high durability, and a material for realizing the device have been requested.

The document WO 2008/120808 A1 (e.g. compound C-5 on page 19) and the document WO2008/120806 A1 disclose polycyclic compounds similar to those described in present claim 1.

### DISCLOSURE OF THE INVENTION

The present invention has been made with a view to solving such problems of the prior art as described above. That is, more specifically, an object of the present invention is to provide a novel fused polycyclic compound and an organic light emitting device having the compound.

The inventors of the present invention have conducted extensive studies, and thus have completed the present invention. That is, the present invention provides a fused polycyclic compound represented by the following general formula (1): 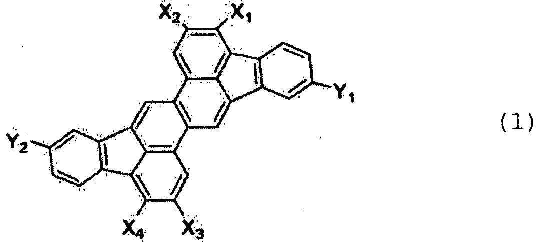 where at least one of X₁ and X₂, at least one of X₃ and X₄, and at least one of Y₁ and Y₂ are each independently selected from an aryl group and a heterocyclic group.

The fused polycyclic compound represented by the general formula (1) of the present invention has been developed based on such a design guideline as described in BEST MODE FOR CARRYING OUT THE INVENTION. In addition, the present invention provides a material for an organic light emitting device having high light emitting efficiency, a high color purity, and high stability, and provides an organic light emitting device having an optical output with extremely high efficiency and an extremely high color purity, and extremely high durability.

Further features of the present invention will become apparent from the following description of exemplary embodiments with reference to the attached drawings.

### BRIEF DESCRIPTION OF THE DRAWINGS

FIG. 1 is a view schematically illustrating organic light emitting devices and units for supplying electrical signals to the organic light emitting devices.
FIG. 2 is a view schematically illustrating a pixel circuit connected to a pixel, and a signal line and a current supply line connected to the pixel circuit.
FIG. 3 is a view illustrating the pixel circuit;
FIG. 4 is a schematic sectional view illustrating an organic light emitting device and a TFT below the device.
FIG. 5 is a view illustrating the structural formula of a fused polycyclic compound C-1, and the electron clouds of its HOMO and LUMO.

### BEST MODE FOR CARRYING OUT THE INVENTION

The fused polycyclic compound represented by the general formula (1) according to the present invention is a compound having a substituent at a specific position of a diindenochrysene skeleton. Unsubstituted diindenochrysene has a fluorescent peak wavelength of 434 nm in a dilute solution, and has such a fluorescent characteristic as to qualify for a blue fluorescent material, or in particular, a light emitting material for an organic light emitting device. The term "blue" refers to a state where the compound has an emission peak in a region of 430 nm to 490 nm, or preferably 430 nm to 445 nm.

Because fused polycyclic compounds each have a planar structure, the compounds are hardly soluble in organic solvents and involve much difficulty upon their synthesis or purification. In view of the foregoing, in the present invention, investigations have been conducted on attempts to avoid those problems by introducing various substituents. Further, when unsubstituted diindenochrysene is used in the emission layer of an organic light emitting device, the following tendency is observed. That is, electroluminescence shifts to longer wavelengths owing to an association between the molecules of the compound depending on the concentration of the compound.

Hereinafter, the effects of the position and kind of a substituent to be introduced on the fluorescent characteristic and thermal stability of a diindenochrysene skeleton are detailed.

Firstly, an effect of a substitution position on a fluorescent wavelength is described. Secondly, a suppressing effect of the introduction of an aryl substituent on the intermolecular association is described. Then, thirdly, the kind of the substituent to be introduced is described.

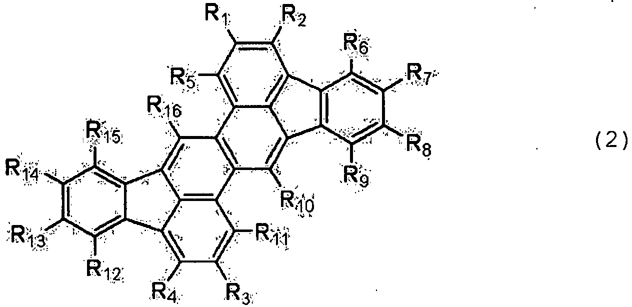

Firstly, the effect of the substitution position on the fluorescent wavelength is described.

The fluorescent spectra of Exemplified Compound A-6 as an example of the general formula (1), and C-1 (diindenochrysene), C-2, C-3, and C-4 as comparison objects in toluene dilute solutions were measured. The structural formula of Exemplified Compound A-6 is shown in the following exemplified compound groups. The structural formulae of C-1, C-2, C-3, and C-4 are shown in comparative examples to be described later.

C-1 is such that R₁ to R₁₆ in the above-mentioned general formula (2) each represent a hydrogen atom. C-2 is such that, in the above-mentioned general formula (2), a 2-methyl-1-naphthyl group is present at each of R₇ and R₁₃, and all other symbols each represent a hydrogen atom. C-3 is such that, in the above-mentioned general formula (2), a phenyl group is present at each of R₆ and R₉, a 3,5-di-tert-butylphenyl group is present at each of R₁₂ and R₁₅, and all other symbols each represent a hydrogen atom. C-4 is such that, in the above-mentioned general formula (2), a 1,3,5-triisopropyl-phenyl group is present at each of R₁ and R₃, and all other symbols each represent a hydrogen atom. As is seen from Table 1 below, neither Exemplified Compound A-6 nor C-4 has a longer wavelength than that of C-1 which is unsubstituted. Based on the results, it can be said that Exemplified Compound A-6 and C-4 each show fluorescence closer to a pure blue color, i.e., 430 nm to 445 nm than each of C-2 and C-3 does, and hence are more excellent blue fluorescent materials than C-2 and C-3 are.

**Table 1 Peak wavelengths of fluorescent spectra in toluene dilute solutions and differences in peak wavelength with C-1**

| | Fluorescent peak wavelength/nm | Difference in peak wavelength with C-1/nm |
|---|---|---|
| C-1 | 434 | - |
| Exemplified Compound A-6 | 439 | 5 |
| C-2 | 447 | 13 |
| C-3 | 456 | 22 |
| C-4 | 437 | 3 |

As described above, an effect of an introduced aryl substituent on a fluorescent spectrum is discussed. Although Exemplified Compound A-6 and C-3 each had four aryl substituents, C-3 had a fluorescent peak wavelength longer than Exemplified Compound A-6 by as large as 17 nm. In addition, C-2 had a fluorescent peak wavelength longer than Exemplified Compound A-6 by 8 nm, though C-2 had two aryl substituents. In view of the foregoing, molecular orbital calculation was conducted on diindenochrysene C-1 at a B3LYP/6-31G* level by employing a density functional theory.

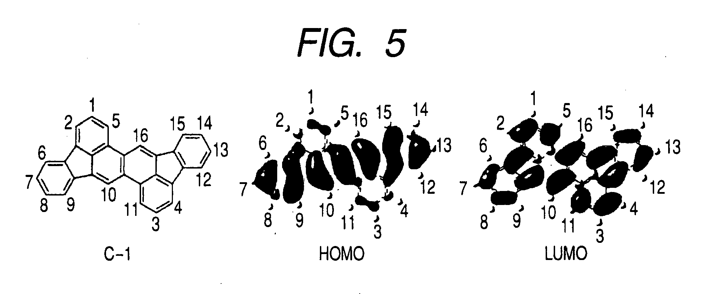

FIG. 5 illustrates the electron clouds of the HOMO and LUMO of the compound C-1.

The results of the molecular orbital calculation showed that the electron clouds of the HOMO were distributed onto carbons bonded to R₁ to R₄, R₈, and R₁₄ in the above-mentioned general formula (2) to a small extent, and were distributed mainly onto carbon atoms except the foregoing. On the other hand, the electron clouds of the LUMO were delocalized in an entire molecule, and in particular, no large biases were observed on carbons to which R₁ to R₁₆ in the above-mentioned general formula (2) were bonded. In consideration of the results of the calculation and the results of the measurement of the fluorescent spectra, it can be understood that the degree of perturbation on an HOMO in a diindenochrysene skeleton varies depending on the position into which a substituent is introduced and the fluorescent characteristic of the skeleton is changed. Therefore, the inventors of the present invention have noticed that a substituent is preferably introduced into such a position as to have no influence on the diindenochrysene skeleton by paying attention to a localized HOMO rather than to a delocalized LUMO.

It can be said that the wavelength of the fluorescent spectrum of each of C-2 having an aryl group at each of R₇ and R₁₃ in the above-mentioned general formula (2) and C-3 having an aryl group at each of R₆, R₉, R₁₂, and R₁₅ in the formula was lengthened by large contribution of resonance stabilization to an HOMO.

Therefore, when a diindenochrysene derivative is used as a blue fluorescent material, the position into which a substituent is introduced is preferably any one of R₁ to R₄, R₈, and R₁₄ in the above-mentioned general formula (2) in order that the lengthening of the wavelength of fluorescence may be prevented.

Secondly, the suppressing effect of the introduction of an aryl substituent on the intermolecular association is described.

The spin-coated films of Exemplified Compound A-6, and C-1, C-2, C-3, and C-4 were produced, and their fluorescent spectra were measured. As a result, it was C-1 that showed the largest peak wavelength shift as compared to that of its fluorescent spectrum in a dilute solution. The fluorescent spectrum in this case was wide and embraced a green to yellow color region. Accordingly, it can be said that C-1 as an unsubstituted product was stabilized by a strong intermolecular association based on a n-electron interaction on its fused polycycle in a solid state, and hence the wavelength of its fluorescence was lengthened.

It can be said that a difference in peak wavelength is small and an intermolecular association is suppressed in each of Exemplified Compound A-6 and C-3 each having four aryl groups, and C-4 having two aryl groups out of the diindenochrysene derivatives each having a substituent.

Therefore, an association between the molecules of a diindenochrysene derivative is suppressed by introducing four aryl groups into diindenochrysene. In addition, when two substituents are introduced, the introduction of an aryl group into each of R₁ and R₃ in the above-mentioned general formula (2) exerts a higher suppressing effect on the intermolecular association than the introduction of an aryl group into each of R₇ and R₁₃ in the above-mentioned general formula (2) does.

Table 2 shows the peak wavelengths of the fluorescent spectra in the spin-coated films and differences in peak wavelength with the fluorescent spectra in the toluene dilute solutions.

**Table 2 Peak wavelengths of fluorescent spectra in spin-coated films and differences in peak wavelength with fluorescent spectra in toluene dilute solutions**

| | Fluorescent peak wavelength/nm | Difference in peak wavelength with fluorescent spectrum in toluene dilute solution/nm |
|---|---|---|
| C-1 | 508 | 74 |
| Exemplified Compound A-6 | 494 | 55 |
| C-2 | 510 | 63 |
| C-3 | 481 | 25 |
| C-4 | 489 | 51 |

Next, a suppressing effect of the introduction of an aryl substituent on an interaction with a host material is described. A co-deposited film was produced by using each of Exemplified Compound A-6, and C-1, C-2, C-3, and C-4 as a dopant material and Compound b-2 shown below as a host material, and its fluorescent spectrum was measured. The dopant material and the host material were deposited from the vapor onto a glass substrate at a weight ratio of 5:95 so as to have a thickness of 20 nm. In addition, it was confirmed that the fluorescent spectrum was caused by the emission of the dopant because the emission of the host material was quenched.

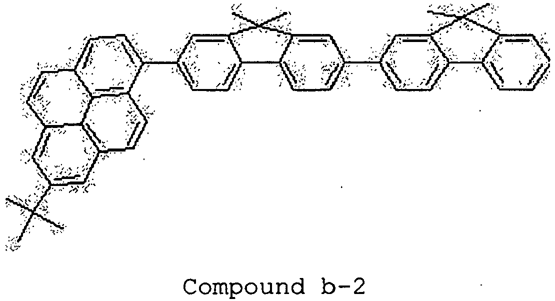

Table 3 shows the peak wavelength and CIE chromaticity coodinates of the fluorescent spectrum in each of the co-deposited films.

It was Exemplified Compound A-6 that was favorable for blue emission in a co-deposited film. In addition, it was also Exemplified Compound A-6 that showed a small change in CIE chromaticity coodinates between a toluene dilute solution and a co-deposited film.

Therefore, four aryl groups are preferably introduced into specific positions, i.e., R₁, R₃, R₈, and R₁₄ in order that good blue emission may be obtained. It can be said that the following facts are reflected in the foregoing. That is, in Exemplified Compound A-6, a suppressing effect on an association between the molecules of Exemplified Compound A-6 is large, and a suppressing effect on an interaction with any other molecule (especially the host material) is large.

**Table 3 CIE chromaticities of fluorescent spectra in co-deposited films and CIE chromaticities in toluene dilute solutions**

| Dopant | CIE chromaticity coodinates of fluorescent spectrum (x, y) Co-deposited film | CIE chromaticity coodinates of fluorescent spectrum (x,y)Toluene dilute solution |
|---|---|---|
| Exemplified Compound A-6 | (0.16, 0.26) | (0.15, 0.17) |
| C-2 | (0.16, 0.32) | (0.14, 0.16) |
| C-3 | (0.15, 0.32) | (0.15, 0.19) |
| C-4 | (0.16, 0.31) | (0.15, 0.12) |

Based on those results, it can be said that a substituent is preferably introduced into such a substitution position that the contribution of resonance stabilization to an HOMO is small in order that a good blue emission spectrum of a compound alone in each of a dilute solution state and a solid film state may be obtained. It can be said that a substituent that hardly causes an intermolecular association or an interaction with the host material is more preferably introduced. To be specific, the substituent preferably substitutes for any one of R₁ to R₄, R₈, and R₁₄ in the general formula (2), and the substitution contributes to the acquisition of a blue emission spectrum favorable for an organic light emitting device. R₁ to R₄, R₈, and R₁₄ each represent preferably an aryl group or a heterocyclic group, or more preferably an aryl group from the viewpoint of small contribution of resonance stabilization with the diindenochrysene skeleton.

Further, each of R₁, R₃, R₈, and R₁₄ in the general formula (2) is preferably substituted in order that a good blue emission spectrum may be obtained even in a solid state mixed or dispersed in any other material. In this case, a suppressing effect on an association between the molecules of the diindenochrysene derivative is large, and a suppressing effect on an interaction with any other molecule (especially the host material) is also large.

The above-mentioned effects lead to a state where an organic light emitting device shows good blue emission when the fused polycyclic compound represented by the general formula (1) is used in its emission layer.

Thirdly, the kind of the substituent to be introduced is described.

The diindenochrysene derivative C-5 in J. Org., 64, 1650-1656(1999) {in the above-mentioned general formula (2), R₁₀ and R₁₆ represent a di(4-tert-butylphenyl)methyl group and all the others represent a hydrogen atom} is a synthesis example and the thermal stability thereof is also described. For example, in "J. Org. Chem., 64, 1650-1656 (1999)", there are descriptions in the main text and "Scheme 5" on pp. 1651-1652, and in the "Chrysene 23" in the "Experimental Section" on page 1654.

According to the document, when C-5, which is originally a yellow solid, is heated in a sealed tube, the color gradually changes to a brown color from 180°C, and the compound melts and decomposes at 332°C. Here, benzyl hydrogen is present in a methyl group bonded to diindenochrysene, and a radical or anion pair produced by its dissociation establishes a resonance structure with the fused polycycle of diindenochrysene so as to be largely stabilized. In addition, the benzyl hydrogen is unstable because three aryl groups are bonded onto an sp³ carbon to which the benzyl hydrogen is bonded so that the groups may be sterically hindered. As described above, the benzyl hydrogen in C-5 is extremely unstable owing to electronic and steric factors, and is assumed to be responsible for the easy thermal decomposition. It is not preferred that C-5 be used as a fluorescent material because such instability may occur not only against heat but also against oxygen, light, a base, or the like.

On the other hand, when Exemplified Compound A-6 was subjected to measurement with a thermogravimetry-differential thermal analysis (TG-DTA) apparatus under a nitrogen atmosphere, no decomposition was observed even at 350°C.

It can be said that the compound has high heat stability because benzyl hydrogen is absent.

Therefore, X₁ to X₉, Y₁, and Y₂ in the general formula (1) for the fused polycyclic compound each represent preferably an aryl group or a heterocyclic group, or more preferably a phenyl group.

The inventors of the present invention have made further extensive studies in view of those three points. As a result, the inventors have found that the following fused polycyclic compound is preferred.

That is, preferred is a fused polycyclic compound represented by the following general formula (1): 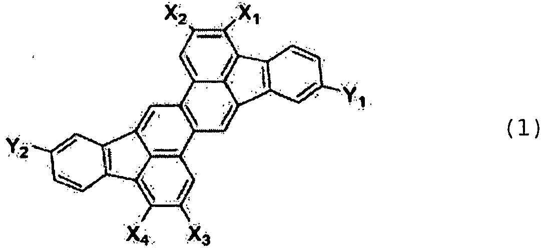 where at least one of X₁ and X₂, at least one of X₃ and X₄, and at least one of Y₁ and Y₂ are each independently selected from an aryl group and a heterocyclic group.

In addition, it is more preferred that X₂, X₃, Y₁, and Y₂ be each independently selected from the aryl group and the heterocyclic group, and X₁ and X₄ each represent a hydrogen atom.

Further, it is still more preferred that X₂, X₃, Y₁, and Y₂ each represent a phenyl group.

There are described aryl groups and heterocyclic groups that the fused polycyclic compound according to the present invention has.

Examples of the aryl groups include a phenyl group, a naphthyl group, a pentalenyl group, an anthryl group, a pyrenyl group, an indacenyl group, an acenaphthenyl group, a phenanthryl group, a phenalenyl group, a fluoranthenyl group, a benzofluoranthenyl group, an acephenanthryl group, an aceanthryl group, a triphenylenyl group, a chrysenyl group, a naphthacenyl group, a perylenyl group, a pentacenyl group, and a fluorenyl group. The examples are, of course, not limited thereto.

Examples of the heterocyclic groups include a pyridyl group, an oxazolyl group, an oxadiazolyl group, a thiazolyl group, a thiadiazolyl group, a carbazolyl group, an acridinyl group, and a phenathrolyl group. The examples are, of course, not limited thereto.

Further, the above-mentioned aryl groups and heterocyclic groups may include those having a substituent. Examples of the substituents that the aryl groups and the heterocyclic groups have include: alkyl groups such as a methyl group, an ethyl group, and a propyl group; aralkyl groups such as a benzyl group and a phenethyl group; aryl groups such as a phenyl group and a biphenylgroup; heterocyclic groups such as a thienyl group, a pyrrolyl group, and a pyridyl group; amino groups such as a dimethylamino group, a diethylamino group, a dibenzylamino group, a diphenylamino group, a ditolylamino group, and a dianisolylamino group; alkoxyl groups such as a methoxyl group, an ethoxyl group, a propoxyl group, and a phenoxyl group; a cyano group; a nitro group; and halogen atoms such as fluorine and chlorine. The examples are, of course, not limited thereto.

An example of the fused polycyclic compound according to the present invention is shown as the following structural formula:

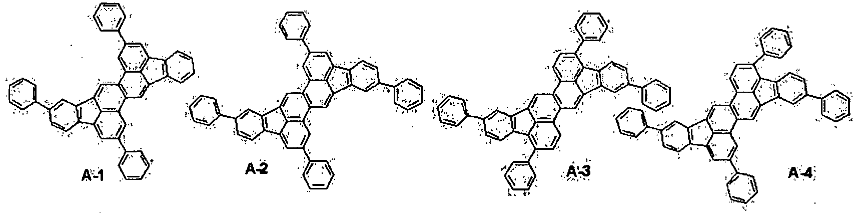

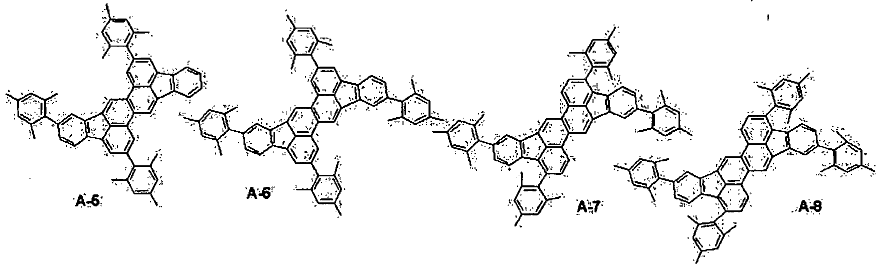

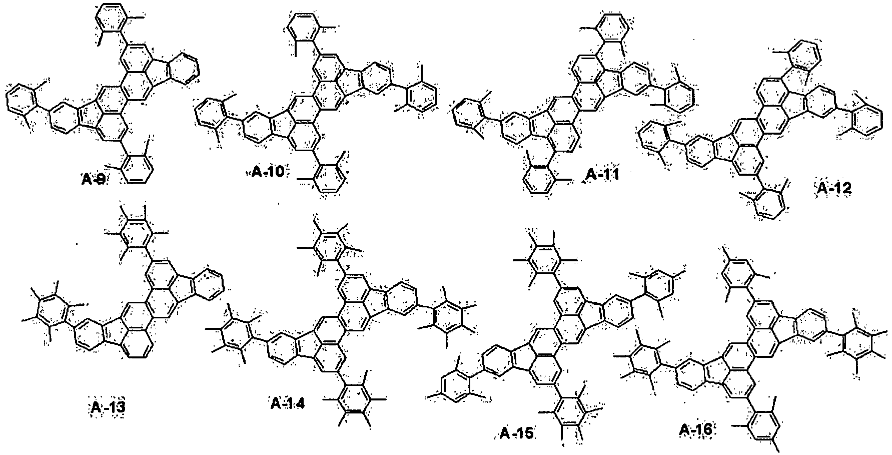

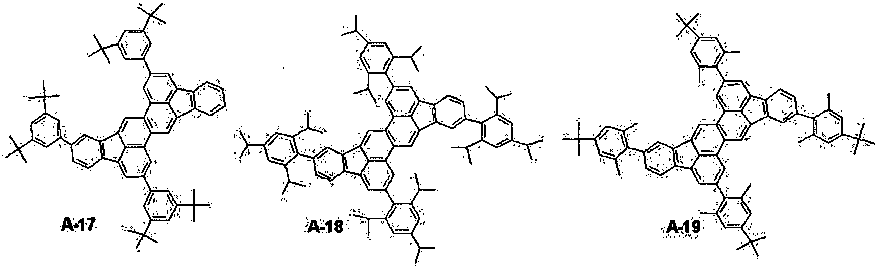

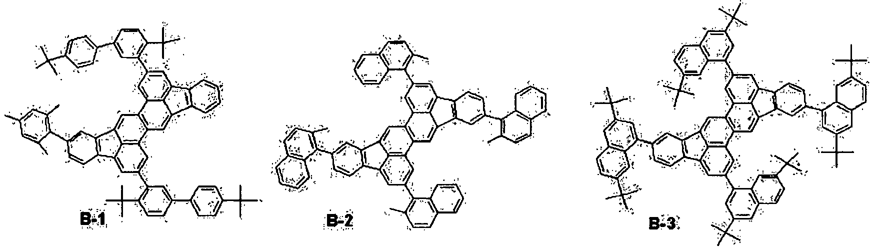

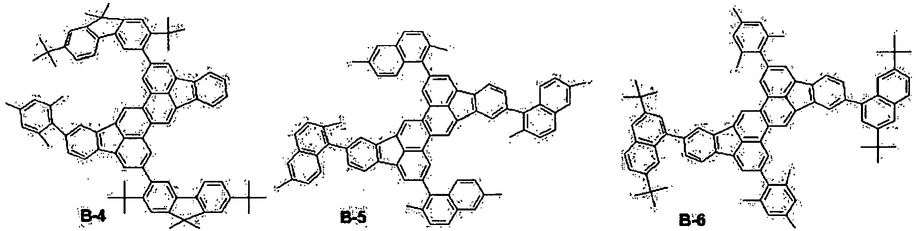

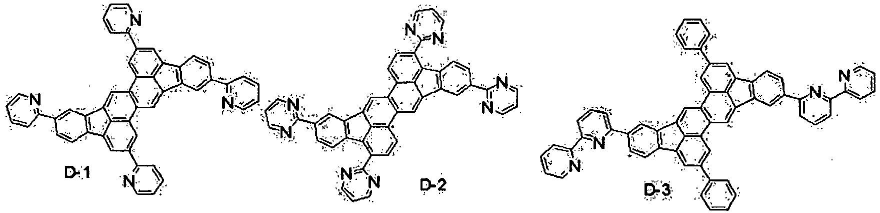

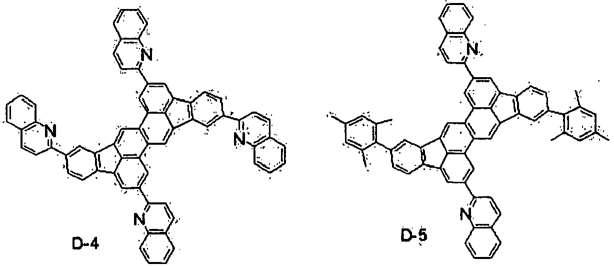

Of the exemplified compounds represented by those structural formulae, A-2, A-6, A-10, A-14, A-15, A-18, A-19, B-2, B-3, B-5, B-6, D-1, D-3, D-4, and D-5 are more preferred because each of the compounds is of such a structure as to have a substituent at each of R₁, R₃, R₈, and R₁₄ in the general formula (2), exerts a large suppressing effect on an association between its molecules and a large suppressing effect on an interaction with any other molecule (especially the host material), and shows good blue emission.

A-2, A-6, A-10, A-14, A-15, A-18, and A-19 are more preferred in terms of heat stability.

A-6, A-10, A-14, A-15, A-18, and A-19 are each more preferred because a phenyl group substituted with an alkyl group substitutes for the diindenochrysene skeleton and hence a suppressing effect on an intermolecular association is significantly large.

A-6 of Example 1 and A-8 of Example 2 are preferred because each of the compounds exerts a large suppressing effect on an association between its molecules and a large suppressing effect on an interaction with any other molecule (especially the host material), shows good blue emission, and has high heat stability. In addition, it is each of A-2, A-3, A-7, A-10, A-11, A-14, A-15, and A-18 out of the exemplified compounds that exerts the same effects.

A-8 of Example 2 is preferred because the compound exerts a large suppressing effect on an association between its molecules and a large suppressing effect on an interaction with any other molecule (especially the host material), shows good blue emission, and has a low sublimation temperature and high heat stability by virtue of its structural asymmetry. In addition, it is each of A-4 and A-12 out of the exemplified compounds that exerts the same effects.

Compound A-16 described in Synthesis Example is preferred because the compound exerts a large suppressing effect on an association between its molecules and a large suppressing effect on an interaction with any other molecule (especially the host material), shows good blue emission, and has a low sublimation temperature and high heat stability by virtue of its asymmetry caused by the presence of two kinds of substituents. In addition, it is A-15 out of the exemplified compounds that exerts the same effects.

Compound B-2 described in Synthesis Example is preferred because the compound exerts a large suppressing effect on an association between its molecules and a large suppressing effect on an interaction with any other molecule (especially the host material), and shows good blue emission. In addition, it is each of Exemplified Compounds B-3 and B-5 that exerts the same effects.

Compound C-1 described in Synthesis Example is preferred because the compound has improved electron-withdrawing property by virtue of the presence of a heterocyclic group as a substituent, has improved oxidation resistance, exerts a large suppressing effect on an association between its molecules and a large suppressing effect on an interaction with any other molecule (especially the host material), and shows good blue emission. In addition, it is each of D-2 and D-4 out of the exemplified compounds represented by those structural formulae that exerts the same effects.

The fused polycyclic compound represented by the general formula (1) can be used as a material for an organic light emitting device.

In the device, the fused polycyclic compound represented by the general formula (1) can be used in each of a hole transport layer, an electron transport layer, and an emission layer. As a result, a device having high light emitting efficiency and a long lifetime can be obtained.

In addition, when the compound represented by the general formula (1) is used in the emission layer, the compound may be used in various modes to obtain a high color purity, high light emitting efficiency, and a long lifetime.

The term "emission layer" refers to a layer that itself emits light. An organic light emitting device according to the present invention may have another functional layer except the emission layer. In this case, the organic light emitting device is such that the emission layer and the other functional layers are laminated. The layer constitution of the organic light emitting device is described later.

An organic compound layer as the emission layer has the fused polycyclic compound represented by the above-mentioned general formula (1).

The emission layer may use the fused polycyclic compound represented by the above-mentioned general formula (1) alone, or may use the compound as a guest material.

The term "guest material" as used in the present invention refers to a material which specifies the substantial emission color of the organic light emitting device and which itself emits light.

The term "host material" refers to a material having a higher composition ratio than that of the guest material.

In the organic emission layer, the guest material has the lower composition ratio and the host material has the higher composition ratio. In this case, each composition ratio is represented in a "wt%" unit by using the total weight of all components of which the organic compound layer is formed as a denominator.

The content of the fused polycyclic compound represented by the above-mentioned general formula (1) to be used as the guest is preferably 0.1 wt% or more to 30 wt% or less with respect to the total weight of the emission layer. The content is more preferably 0.1 wt% or more to 15 wt% or less when concentration quenching is suppressed. Any such numerical range holds true even for the case where the organic compound layer is formed only of the host material and the guest material.

In the organic compound layer, the guest material may be uniformly incorporated into the entirety of the organic compound layer, or may be incorporated so as to have a concentration gradient. Alternatively, the guest material may be incorporated only into a certain region of the organic compound layer, and another region free of the guest material may be present.

In addition, when the fused polycyclic compound represented by the above-mentioned general formula (1) is used as the guest, the host material is not particularly limited. A fused polycyclic derivative is preferably used in order that an organic light emitting device formed of a stable amorphous film may be provided. In addition, the host material itself is requested to have a high light emitting yield and chemical stability in order that an organic light emitting device having high efficiency and high durability may be provided. Accordingly, a fused polycyclic derivative which has a high fluorescent quantum yield and which is chemically stable, such as a fluorene derivative, a pyrene derivative, a fluoranthene derivative, or a benzofluoranthene derivative, is more preferred.

In order that an organic light emitting device having durability may be provided, a compound for an organic light emitting device of which the device is formed must have chemical stability.

The fused polycyclic compound represented by the above-mentioned general formula (1) has low reactivity based on an electrophilic reaction of a singlet oxygen molecule or the like by virtue of an electron-withdrawing effect of a five-membered ring structure. As a result, the compound is chemically stable. In addition, the skeleton of the compound, which has two five-membered ring structures, has higher chemical stability than a skeleton having one five-membered ring structure such as fluoranthene or benzofluoranthene.

The fused polycyclic compound represented by the above-mentioned general formula (1) has electron injection property by virtue of the electron-withdrawing property of each five-membered ring structure. As a result, the compound can reduce the voltage at which an organic light emitting device is driven when the compound is used as a material for the device. In addition, the skeleton having two five-membered ring structures exerts a higher reducing effect on the voltage at which the device is driven than a skeleton having one five-membered ring structure such as fluoranthene or benzofluoranthene.

When the organic light emitting device according to the present invention is applied to a display, the device can be preferably used as a blue emission pixel in the display region of the display. The fused polycyclic compound represented by the above-mentioned general formula (1) in a dilute solution shows an emission peak at 430 to 450 nm, which is an optimum peak position for blue emission.

Next, the organic light emitting device of the present invention is described in detail.

The organic light emitting device of the present invention includes a pair of electrodes, an anode and a cathode, and one of a single layer and a plurality of layers which include an organic compound and are interposed between the pair of electrodes, and at least one layer of the layers containing the organic compound contains at least one kind of fused polycyclic compound represented by the general formula (1).

At least one layer containing an organic compound, i.e., organic compound layer is provided in the organic light emitting device according to the present invention.

The device may have a compound layer except the above-mentioned organic compound layer between the pair of electrodes.

Alternatively, two or more compound layers including the organic compound layer may be provided between the pair of electrodes, and the device in such case is called a multilayer organic light emitting device.

Hereinafter, preferred examples of the multilayer organic light emitting device, i.e., first to fifth examples are described.

An organic light emitting device of such a constitution that the anode, an emission layer, and the cathode are sequentially provided on a substrate can be given as the first example of the multilayer organic light emitting device. The organic light emitting device used here is useful when a compound that itself has hole transport property, electron transport property, and emission property alone is used or when compounds having the respective properties are used as a mixture.

An organic light emitting device of such a constitution that the anode, a hole transport layer, an electron transport layer, and the cathode are sequentially provided on a substrate can be given as the second example of the multilayer organic light emitting device. This case is useful when a material having one or both of hole transport property and electron transport property is used as an emission substance in each layer in combination with a mere hole transport substance or electron transport substance having no emission property. In addition, in this case, the emission layer is formed of any one of the hole transport layer and the electron transport layer.

An organic light emitting device of such a constitution that the anode, the hole transport layer, the emission layer, the electron transport layer, and the cathode are sequentially provided on a substrate can be given as the third example of the multilayer organic light emitting device. The device constitution is such that a carrier transport function and an emission function are separated from each other. In addition, the constitution can be used in combination with compounds having the respective properties, i.e., hole transport property, electron transport property, and emission property at appropriate times. In addition, as the degree of freedom in material selection increases to an extreme extent, various compounds having different emission wavelengths can be used. As a result, the diversification of emission hues can be achieved. Further, each carrier or exciton is effectively trapped in the central emission layer, and hence an improvement in emission efficiency can also be achieved.

An organic light emitting device of such a constitution that the anode, a hole injection layer, the hole transport layer, the emission layer, the electron transport layer, and the cathode are sequentially provided on a substrate can be given as the fourth example of the multilayer organic light emitting device. The device constitution has an improving effect on adhesiveness between the anode and the hole transport layer or on hole injection property, and is effective in reducing the voltage at which the device is driven.

An organic light emitting device of such a constitution that the anode, the hole transport layer, the emission layer, a hole/exciton blocking layer, the electron transport layer, and the cathode are sequentially provided on a substrate can be given as the fifth example of the multilayer organic light emitting device. The constitution is such that a layer for blocking the escape of a hole or exciton toward the cathode (the hole/exciton blocking layer) is inserted between the emission layer and the electron transport layer. The use of a compound having an extremely high ionization potential as the hole/exciton blocking layer is a constitution effective in improving the emission efficiency.

An emission region containing the fused polycyclic compound represented by the general formula (1) in the present invention is the region of the emission layer described above.

However, the first to fifth examples of the multilayer organic light emitting device have only a basic device constitution, and the constitution of the organic light emitting device using the fused polycyclic compound according to the present invention is not limited thereto. There can be given various layer constitutions, for example, a constitution in which an insulating layer is provided at an interface of an electrode and an organic layer, an adhesive layer or an interference layer is provided, or an electron transport layer or a hole transport layer is formed of two layers having different ionization potentials.

The fused polycyclic compound represented by the general formula (1) according to the present invention can be used in any of the forms of from the first to fifth examples.

In the organic light emitting device according to the present invention, at least one kind of organic compound represented by the general formula (1) according to the present invention is incorporated into the layer containing an organic compound, and the compound is particularly preferably used as a dopant material for the emission layer.

The fused polycyclic compound according to the present invention may be used as a host material for the emission layer.

The fused polycyclic compound according to the present invention may be used in at least one of the respective layers except the emission layer, i.e., the hole injection layer, the hole transport layer, the hole/exciton blocking layer, the electron transport layer, and the electron injection layer.

Here, there can be used together a conventionally known compound as required, in addition to the fused polycyclic compound of the present invention, such as a low-molecular or high-molecular hole transport compound, a light emitting compound, an electron transport compound, or the like.

A hole injection/transport material is preferably a material having a high hole mobility to facilitate the injection of a hole from an anode and to transport the injected hole to an emission layer. As low-molecular and high-molecular materials having hole injection/transport properties, there are exemplified a triarylamine derivative, a phenylenediamine derivative, a stilbene derivative, a phthalocyanine derivative, a porphyrin derivative, poly(vinylcarbazole), poly(thiophene), and other conductive polymers, but the material is of course not limited thereto.

As a host material, there are exemplified: fused ring compounds (such as a fluorene derivative, a naphthalene derivative, an anthracene derivative, a pyrene derivative, a carbazole derivative, a quinxaline derivative, and a quinoline derivative); organic aluminum complexes such as tris(8-quinolinolato)aluminum; organic zinc complexes; and polymer derivatives such as a triphenylamine derivative, a poly(fluorene) derivative, and a poly(phenylene) derivative, but the material is of course not limited thereto.

The electron injection/transport material may be arbitrarily selected from compounds each of which facilitates the injection of an electron from a cathode and is capable of transporting the injected electron to the emission layer. In addition, the material is selected in consideration of, for example, a balance with the hole mobility of the hole injection/transport material. As materials having electron injection/transport properties, there are exemplified an oxadiazole derivative, an oxazole derivative, a pyrazine derivative, a triazole derivative, a triazine derivative, a quinoline derivative, a quinoxaline derivative, a phenanthroline derivative, and organic aluminum complexes, but the material is of course not limited thereto.

As an anode material, a material having as large a work function as possible is preferred. Examples of the material which may be used include: metal elements such as gold, platinum, silver, copper, nickel, palladium, cobalt, selenium, vanadium, and tungsten, and alloys of those metal elements; and metal oxides such as tin oxide, zinc oxide, indium oxide, indium tin oxide (ITO), and indium zinc oxide. Further, conductive polymers such as polyaniline, polypyrrole, and polythiophene may also be used. Each of those electrode substances may be used alone, or a plurality of kinds thereof may be used in combination. Further, the anode may be formed of a single layer, or may be formed of multiple layers.

On the other hand, as a cathode material, a material having a small work function is preferred. Examples of the material include: alkali metals such as lithium; alkali earth metals such as calcium; and metal elements such as aluminum, titanium, manganese, silver, lead, and chromium. Alternatively, alloys including a combination of those metal elements may also be used. For example, magnesium-silver, aluminum-lithium, and aluminum-magnesium can be used. Metal oxides such as indium tin oxide (ITO) may also be used. One kind of those electrode substances may be used alone, or a plurality of kinds thereof may be used in combination. Further, the cathode may be formed of a single layer, or may be formed of multiple layers.

Examples of the substrate having the organic light emitting device according to the present invention include, but are not particularly limited to: opaque substrates such as metallic substrates and ceramic substrates; and transparent substrates such as glass, quartz, and plastic sheet substrates. In addition, a color filter film, a fluorescent color conversion filter film, a dielectric reflection film, or the like may be used in the substrate to control emission colors.

It should be noted that a protective layer or a sealing layer may be formed on the prepared device to prevent the device from contacting oxygen, moisture, or the like. Examples of the protective layer include a diamond thin film, a film made of an inorganic material such as a metal oxide or a metal nitride, a polymer film made of a fluorine resin, polyethylene, a silicone resin, a polystyrene resin, or the like, and a photo-curing resin. Further, the device itself can be covered with glass, a gas-impermeable film, a metal, or the like and packaged with an appropriate sealing resin.

In the organic light emitting device according to the present invention, a layer containing the organic compound according to the present invention and a layer formed of another organic compound are formed by a method described below. In general, a thin film is formed by a vacuum deposition method, an ionization-assisted deposition method, a sputtering method, or a plasma method, or the thin film may be formed by dissolving the compound in a suitable solvent and subjecting the resultant to a known coating method (e.g., a spin coating method, a dipping method, a casting method, an LB method, or an ink jet method). Here, when the layer is formed by the vacuum deposition method, a solution coating method, or the like, the layer hardly undergoes crystallization or the like, and is excellent in stability over time. In addition, in film formation by the coating method, the film may be formed by using a compound in combination with an appropriate binder resin.

Examples of the above-mentioned binder resin include, but are not limited to, a polyvinylcarbazole resin, a polycarbonate resin, a polyester resin, an ABS resin, an acrylic resin, a polyimide resin, a phenol resin, an epoxy resin, a silicone resin, and a urea resin. In addition, as a homopolymer or a copolymer, one kind of binder resin may be used alone or a mixture of two or more kinds may be used. Further, a known additive such as a plasticizer, an antioxidant, or a UV absorber, as required, may be used in combination.

The organic light emitting device according to the present invention can be applied to products which require energy saving and high luminance. Examples of the applications include a display apparatus, a lighting apparatus, a light source of a printer, and a backlight of a liquid crystal display apparatus.

As the display apparatus, an energy-saving, lightweight flat panel display having high visibility can be produced. The display apparatus can be used as an image display apparatus such as a PC, a television, or an advertising medium. Alternatively, the display apparatus may be used in the display portion of an imaging apparatus such as a digital still camera or a digital video camera.

Alternatively, the display apparatus may be used in the operation display portion of an electrophotographic image-forming apparatus, that is, a laser beam printer, copying machine, or the like.

In addition, the display apparatus can be used as a light source used upon exposure of a latent image to the photosensitive member of an electrophotographic image-forming apparatus, that is, a laser beam printer, copying machine, or the like. Multiple organic light emitting devices that can be independently addressed are placed in an array fashion (such as a line fashion), and the photosensitive drum is subjected to desired exposure. As a result, the latent image can be formed. The use of the organic light emitting device according to the present invention can reduce a space that has been conventionally needed for placing a light source, a polygon mirror, various optical lenses, and the like.

As for the lighting apparatus and the backlight, an energy-saving effect can be expected by using the organic light emitting device of the present invention. Further, the organic light emitting device of the present invention can be used as a planar light source.

In addition, an emission color can be controlled by providing the substrate supporting the organic light emitting device according to the present invention with a color filter film, a fluorescent color conversion filter film, a dielectric reflection film, or the like. In addition, whether or not the organic light emitting device emits light can be controlled by providing the substrate with a thin-film transistor (TFT), and connecting the device to the TFT. In addition, multiple organic light emitting devices are arranged in a matrix fashion, i.e., in an in-plane direction, and the resultant can be used as a lighting apparatus.

Next, a display apparatus using the organic light emitting device according to the present invention is described. The display apparatus includes the organic light emitting device according to the present invention and units for supplying electrical signals to the organic light emitting device according to the present invention. Hereinafter, the display apparatus according to the present invention is described in detail with reference to the drawings by taking an active matrix type as an example.

First, the symbols in FIGS are outlined. A display apparatus is represented by 1, pixel circuits are each represented by 2 and 15, a scanning signal driver is represented by 11, an information signal driver is represented by 12, a current supply source is represented by 13, and a pixel is represented by 14. A first thin-film transistor (TFT1) is represented by 21, a capacitor (C_{add}) is represented by 22, a second thin-film transistor (TFT2) is represented by 23, and an organic light emitting device is represented by 24.

A substrate is represented by 31, a moisture-proof layer is represented by 32, a gate electrode is represented by 33, a gate insulating film is represented by 34, a semiconductor film is represented by 35, a drain electrode is represented by 36, a source electrode is represented by 37, a TFT device is represented by 38, and an insulating film is represented by 39.

As contact hole (through hole) is represented by 310, an anode is represented by 311, an organic layer is represented by 312, a cathode is represented by 313, a first protective layer is represented by 314, and a second protective layer is represented by 315.

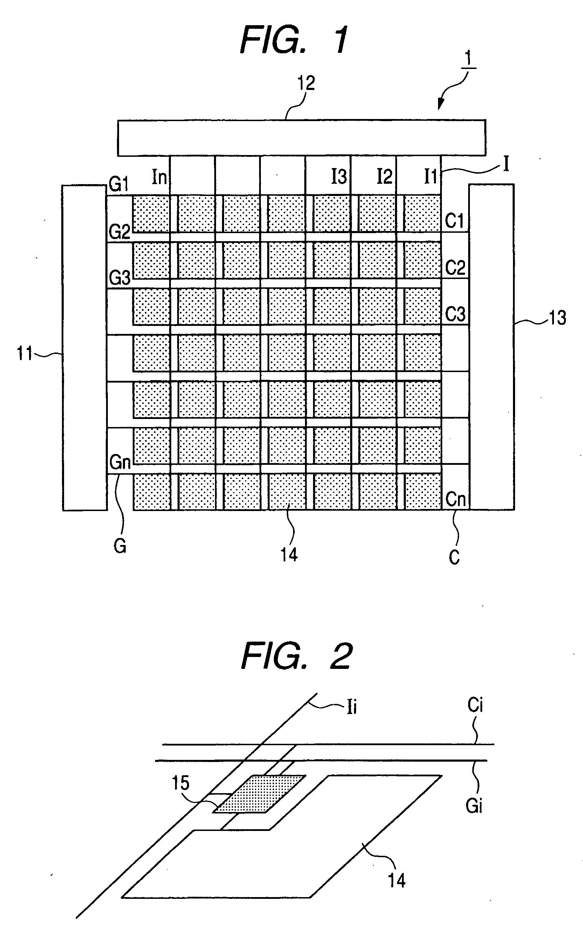

FIG. 1 illustrates one form of the display apparatus. The figure schematically illustrates an example of the constitution of the display apparatus including the organic light emitting device according to the present invention and the units for supplying electrical signals to the organic light emitting device according to the present invention.

FIG. 2 is a view schematically illustrating a pixel circuit connected to a pixel, and a signal line and a current supply line connected to the pixel circuit.

The units for supplying electrical signals to the organic light emitting device according to the present invention refer to the scanning signal driver 11, the information signal driver 12, and the current supply source 13 in FIG. 1, and the pixel circuit 15 in FIG. 2.

In the display apparatus 1 of FIG. 1, the scanning signal driver 11, the information signal driver 12, and the current supply source 13 are placed, and are connected to gate selection lines G, information signal lines I, and current supply lines C, respectively. The pixel circuits 15 are placed at points of intersection of the gate selection lines G and the information signal lines I (FIG. 2). The pixels 14 each formed of the organic light emitting device according to the present invention are provided in correspondence with the pixel circuits 15. The pixels 14 are each an organic light emitting device. Therefore, each organic light emitting device is illustrated as an emission point in the figure. In the figure, the upper electrode of one organic light emitting device may be common to the upper electrode of another organic light emitting device. Of course, an upper electrode may be individually provided for each light emitting device.

The scanning signal driver 11 sequentially selects the gate selection lines G1, G2, G3, ···, Gn, and in synchronization with the selection, an image signal is applied from the information signal driver 12 to each of the pixel circuits 15 through any one of the information signal lines I1, I2, I3, ···, In.

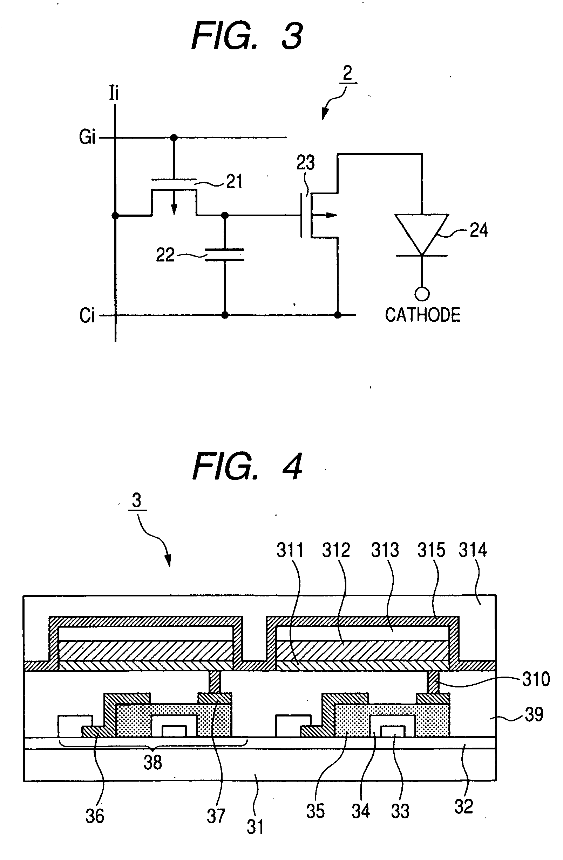

Next, the operation of each pixel is described. FIG. 3 is a circuit diagram illustrating a circuit of which one pixel placed in the display apparatus of FIG. 1 is formed. In FIG. 3, the second thin-film transistor (TFT2) 23 controls a current for causing an organic light emitting device 24 to emit light. In the pixel circuit 2 of FIG. 3, when a selection signal is applied to the gate selection line Gi, the first thin-film transistor (TFT1) 21 is turned on, and an image signal Ii is supplied to the capacitor (C_{add}) 22 so that the gate voltage of the second thin-film transistor (TFT2) 23 may be determined. A current is supplied from the current supply line Ci to the organic light emitting device 24 in accordance with the gate voltage of the second thin-film transistor (TFT2) (23). Here, the gate potential of the second thin-film transistor (TFT2) 23 is held in the capacitor (C_{add}) 22 until the first thin-film transistor (TFT1) 21 is selected for next scan. Accordingly, a current continues to flow in the organic light emitting device 24 until the next scan is performed. As a result, the organic light emitting device 24 can be caused to emit light at all times during a one-frame period.

It should be noted that the organic light emitting device according to the present invention can be used also in a voltage writable display apparatus in which a thin-film transistor controls a voltage between the electrodes of the organic light emitting device 24, though illustration is omitted.

FIG. 4 is a schematic view illustrating an example of the sectional structure of a TFT substrate used in the display apparatus of FIG. 1. Details about the structure are described below while an example of a production process for the TFT substrate is shown.

Upon production of a display apparatus 3 of FIG. 4, first, the upper portion of the substrate 31 such as glass is coated with the moisture-proof film 32 for protecting a member to be formed on the substrate (a TFT or an organic layer). Silicon oxide, a composite of silicon oxide and silicon nitride, or the like is used as a material of which the moisture-proof film 32 is formed. Next, a metal such as Cr is formed into a film by sputtering, and the film is patterned into a predetermined circuit shape. Thus, the gate electrode 33 is formed.

Subsequently, silicon oxide or the like is formed into a film by, for example, a plasma CVD method or a catalytic chemical vapor deposition method (cat-CVD method), and the film is patterned. Thus, the gate insulating film 34 is formed. Next, a silicon film is formed by a plasma CVD method or the like (annealing is performed at a temperature of 290°C or higher in some cases), and the film is patterned in accordance with the circuit shape. Thus, the semiconductor film 35 is formed.

Further, the semiconductor film 35 is provided with the drain electrode 36 and the source electrode 37 so that the TFT device 38 may be produced. Thus, such circuit as illustrated in FIG. 3 is formed. Next, the insulating film 39 is formed on the upper portion of the TFT device 38. Next, the contact hole (through-hole) 310 is formed so that the anode 311 for an organic light emitting device formed of a metal and the source electrode 37 may be connected to each other.

The one or multiple organic layers 312 and the cathode 313 are sequentially laminated on the anode 311. As a result, the display apparatus 3 can be obtained. In this case, the first protective layer 314 and the second protective layer 315 may be provided for preventing the deterioration of an organic light emitting device. When the display apparatus using the organic light emitting device of the present invention is driven, the display apparatus can achieve display which has good image quality and which is stable over a long time period.

It should be noted that in the above-mentioned display apparatus, a switching device is not particularly limited, and a single crystal silicon substrate, MIM device, a-Si type device, or the like can be easily applied to the apparatus.

One or multiple organic emission layers and a cathode layer are sequentially laminated on the above-mentioned ITO electrode. As a result, an organic light emitting display panel can be obtained. When the display panel using the fused polycyclic compound of the present invention is driven, the display panel can achieve display which has good image quality and which is stable over a long time period.

Moreover, with respect to a direction of extracting light from the device, both a bottom emission structure (structure in which light is extracted from the substrate side) and a top emission structure (structure in which light is extracted from a side opposite to the substrate) may be adopted.

Hereinafter, the present invention is described more specifically by way of examples. However, the present invention is not limited to the examples.

### (Example 1)

### (Synthesis of Exemplified Compound A-6)

Synthesis was performed in accordance with the following scheme.

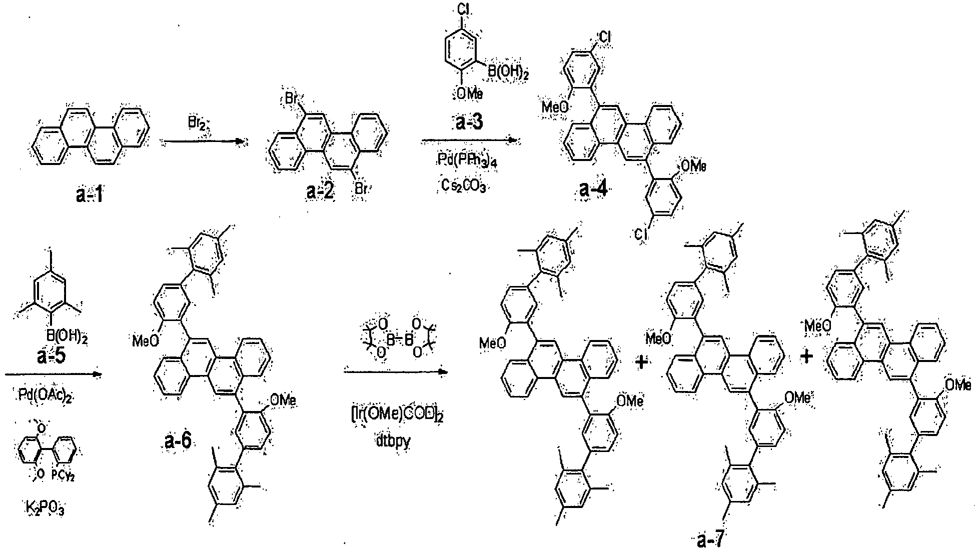

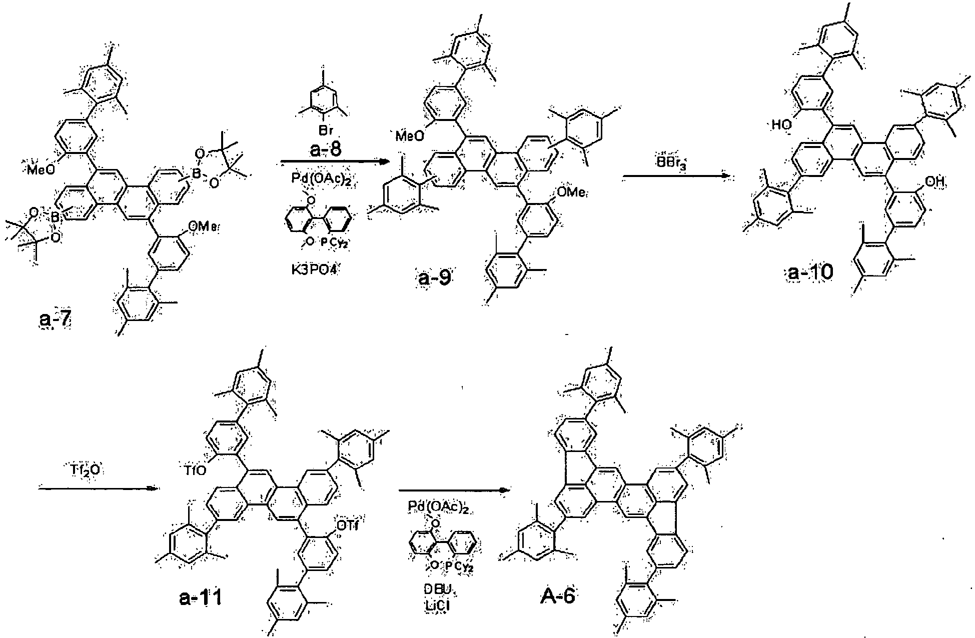

### (a) Synthesis of Compound a-2

First, 15.16 g (66.4 mmol) of chrysene (Compound a-1) and 350 ml of carbon tetrachloride were loaded into a 500-ml three-necked flask. While the mixture was stirred at room temperature, 21 g (131 mmol) of bromine were slowly dropped to the mixture over 100 minutes. The temperature of the reaction solution was increased, and then the reaction solution was heat-refluxed for 3 hours. The reaction solution was cooled to room temperature, and the precipitated crystal was filtrated. The resultant crystal was recrystallized with a toluene solvent. As a result, 19.5 g of Compound a-2 (white crystal) were obtained (in 76.1% yield).

### (b) Synthesis of Compound a-4

First, 2.69 g (7.00 mmol) of Compound a-2, 3.73 g (20.0 mmol) of Compound a-3, 20 ml of toluene, and 10 ml of ethanol were loaded into a 100-ml three-necked flask. While the mixture was stirred at room temperature in a nitrogen atmosphere, an aqueous solution of 10 g of cesium carbonate in 20 ml of water was dropped to the mixture, and then 81 mg of tetrakis(triphenylphosphine)palladium(0) were added to the mixture. The temperature of the resultant mixture was increased to 77°C, and then the mixture was stirred for 5 hours. After the reaction, the organic layer was extracted with toluene and dried with anhydrous sodium sulfate. After that, the dried product was purified with a silica gel column (using a mixture of toluene and heptane as a developing solvent). As a result, 3.20 g of Compound a-4 (white crystal) were obtained (in 89% yield).

### (c) Synthesis of Compound a-6

First, 3.20 g (1.65 mmol) of Compound a-4, 3.10 g (18.9 mmol) of Compound a-5, 141 mg (0.630 mmol) of palladium acetate, 13.4 g (63.0 mmol) of potassium phosphate, 621 mg (1.51 mmol) of 2-dicyclohexylphosphino-2'-6'-dimethoxybiphenyl, 80 ml of toluene, and 5 ml of water were loaded into a 50-ml three-necked flask. While the mixture was stirred in a nitrogen atmosphere, the temperature of the resultant mixture was increased to 90°C, and then the mixture was stirred for 6 hours. After the reaction, 100 ml of water were added, followed by a reaction, the organic layer was extracted with toluene and dried with anhydrous sodium sulfate. After that, the dried product was purified with a silica gel column (using toluene as a developing solvent). As a result, 2.70 g of Compound a-6 (whitish yellow crystal) were obtained (in 63.3% yield).

### (d) Synthesis of Compound a-7

First, 1.30 g (1.92 mmol) of Compound a-6, 0.98 g (3.84 mmol) of bis(pinacolato)diboron, 254 mg (0.384 mmol) of [Ir(OMe)COD]₂, 0.268 g (1.0 mmol) of 4,4'-di-tert-butyl-2,2'-bipyridine (dtbpy), and 50 ml of cyclohexane were loaded into a 200-ml three-necked flask. In a nitrogen atmosphere, the temperature of the mixture was increased to 80°C, and then the mixture was stirred for 5 hours. After the reaction, the organic layer was extracted with chloroform and dried with anhydrous sodium sulfate. After that, the dried product was purified with a silica gel column (using chloroform as a developing solvent). As a result, 1.53 g of Compound a-7 (white crystal) as a mixture of three kinds of isomers of a pinacolborane product were obtained (in 86% yield).

### (e) Synthesis of Compound a-9

First, 3.20 g (3.45 mmol) of Compound a-7, 2.06 g (10.34 mmol) of Compound a-8, 77.5 mg (0.345 mmol) of palladium acetate, 7.32 g (34.5 mmol) of potassium phosphate, 311 mg (0.759 mmol) of 2-dicyclohexylphosphino-2'-6'-dimethoxybiphenyl, 50 ml of dioxane, and 5 ml of water were loaded into a 100-ml three-necked flask. In a nitrogen atmosphere, the temperature of the mixture was increased to 100°C, and then the mixture was stirred for 6 hours. After the reaction, 100 ml of water were added to the reaction solution, and the organic layer was extracted with toluene and dried with anhydrous sodium sulfate. After that, the dried product was purified with a silica gel column (using toluene as a developing solvent). As a result, 1.60 g of Compound a-9 (whitish yellow crystal) as a mixture of three kinds of isomers of a mesityl group substitution product were obtained (in 50.8% yield).

### (f) Synthesis of Compound a-10

First, 1.0 g (1.10 mmol) of Compound a-9 and 50 ml of dichloromethane were loaded into a 100-ml three-necked flask. While the mixture was stirred under ice cooling in a nitrogen atmosphere, 3.30 ml of boron tribromide were slowly dropped to the mixture. After having been stirred for 1 hour, the reaction solution was stirred at room temperature for 8 hours. After the reaction, 50 ml of water were added to the reaction solution, and the organic layer was extracted with chloroform and dried with anhydrous sodium sulfate. After that, the dried product was purified with a silica gel column (using a mixture of chloroform and ethyl acetate as a developing solvent). As a result, 0.450 g of Compound a-10 (whitish yellow crystal) was obtained (in 47% yield). Isomers were separated at this time point.

### (g) Synthesis of Compound a-11

First, 0.450 g (0.517 mmol) of Compound a-10 and 50 ml of anhydrous pyridine were loaded into a 100-ml three-necked flask. While the mixture was stirred under ice cooling in a nitrogen atmosphere, 0.376 ml (2.07 mmol) of trifluoromethanesulfonic anhydride (Tf₂O) were slowly dropped to the mixture. After having been stirred for 1 hour, the reaction solution was stirred at room temperature for 2 hours. After the reaction, 50 ml of water were added to the reaction solution, and the organic layer was extracted with toluene and dried with anhydrous sodium sulfate. After that, the dried product was purified with a silica gel column (using a mixture of chloroform and heptane as a developing solvent). As a result, 0.386 g of Compound a-11 (whitish yellow crystal) was obtained (in 65% yield).

### (h) Synthesis of Exemplified Compound A-6

First, 0.320 g (0.278 mmol) of Compound a-11, 125 mg (0.557 mmol) of palladium acetate, 1.6 ml of diazabicycloundecene (DBU), 70.8 mg (1.66 mmol) of lithium chloride, 502 mg (1.22 mmol) of 2-dicyclohexylphosphino-2'-6'-dimethoxybiphenyl, and 20 ml of DMF were loaded into a 50-ml three-necked flask. In a nitrogen atmosphere, the mixture was stirred at room temperature. After that, the temperature of the mixture was increased to 150°C, and then the mixture was stirred for an additional 5 hours. After the reaction, the organic layer was extracted with chloroform and dried with anhydrous sodium sulfate. After that, the dried product was purified with a silica gel column (using a mixture of toluene and heptane as a developing solvent). As a result, 60 mg of Exemplified Compound A-6 (yellow crystal) were obtained (in 25.4% yield).

Mass spectrometry confirmed that Exemplified Compound A-6 had an M+ of 849.

Further, the structure of Exemplified Compound A-6 was confirmed by ¹H-NMR measurement.

¹H-NMR (CDCl₃, 400 MHz) σ (ppm): 9.14 (s, 2H), 8.44 (s, 2H), 7.97 (d, 2H), 7.87 (m, 4H), 7.21 (d, 2H), 7.05 (s, 2H), 7.05 (s, 2H), 6.98 (s, 2H), 6.98 (s, 2H), 2.40 (s, 6H), 2.37 (s, 6H), 2.14 (s, 12H), 2.12 (s, 12H)

The fluorescent spectrum of a toluene solution containing Exemplified Compound A-6 at a concentration of 1×10⁻⁵ mol/l was measured with F-4500 manufactured by Hitachi, Ltd. at an excitation wavelength of 370 nm. The fluorescent peak wavelength is as shown in Table 1 above.

Further, a tetrahydrofuran solution containing Exemplified Compound A-6 at a concentration of 0.1 wt% was prepared. The solution was dropped onto a glass plate. After that, the solution was subjected to spin coating initially at a revolution speed of 500 RPM for 10 seconds and then at a revolution speed of 1000 RPM for 40 seconds. Thus, a film was formed. Next, the fluorescent spectrum of the above-mentioned organic film was measured with F-4500 manufactured by Hitachi, Ltd. at an excitation wavelength of 370 nm. The fluorescent peak wavelength is as shown in Table 2 above.

Exemplified Compound A-6 and Compound b-2 were deposited from the vapor onto a glass substrate at a weight ratio of 5:95 so as to have a thickness of 20 nm. In addition, it was confirmed that the fluorescent spectrum was caused by the emission of the dopant because the emission of the host material was quenched. The CIE chromaticity coodinates is as shown in Table 3 above.

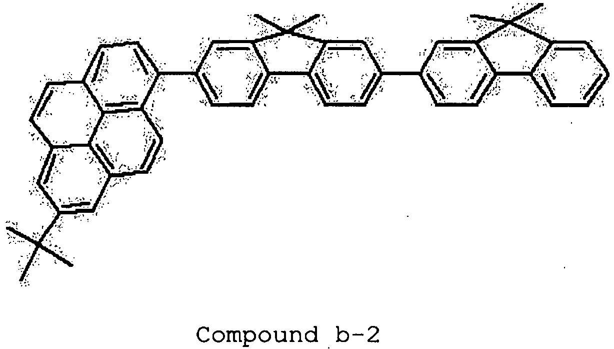

### (Example 2)

### (Synthesis of Exemplified Compounds A-7 and A-8)

The process up to the synthesis of Compound a-9 was performed in the same manner as in Example 1. OH products as intermediates for A-7 and A-8 which could be isolated at the time of the purification of Compound a-10 in the section (f) of Example 1 were each isolated. Then, Exemplified Compounds A-7 and A-8 were synthesized in accordance with the following schemes.

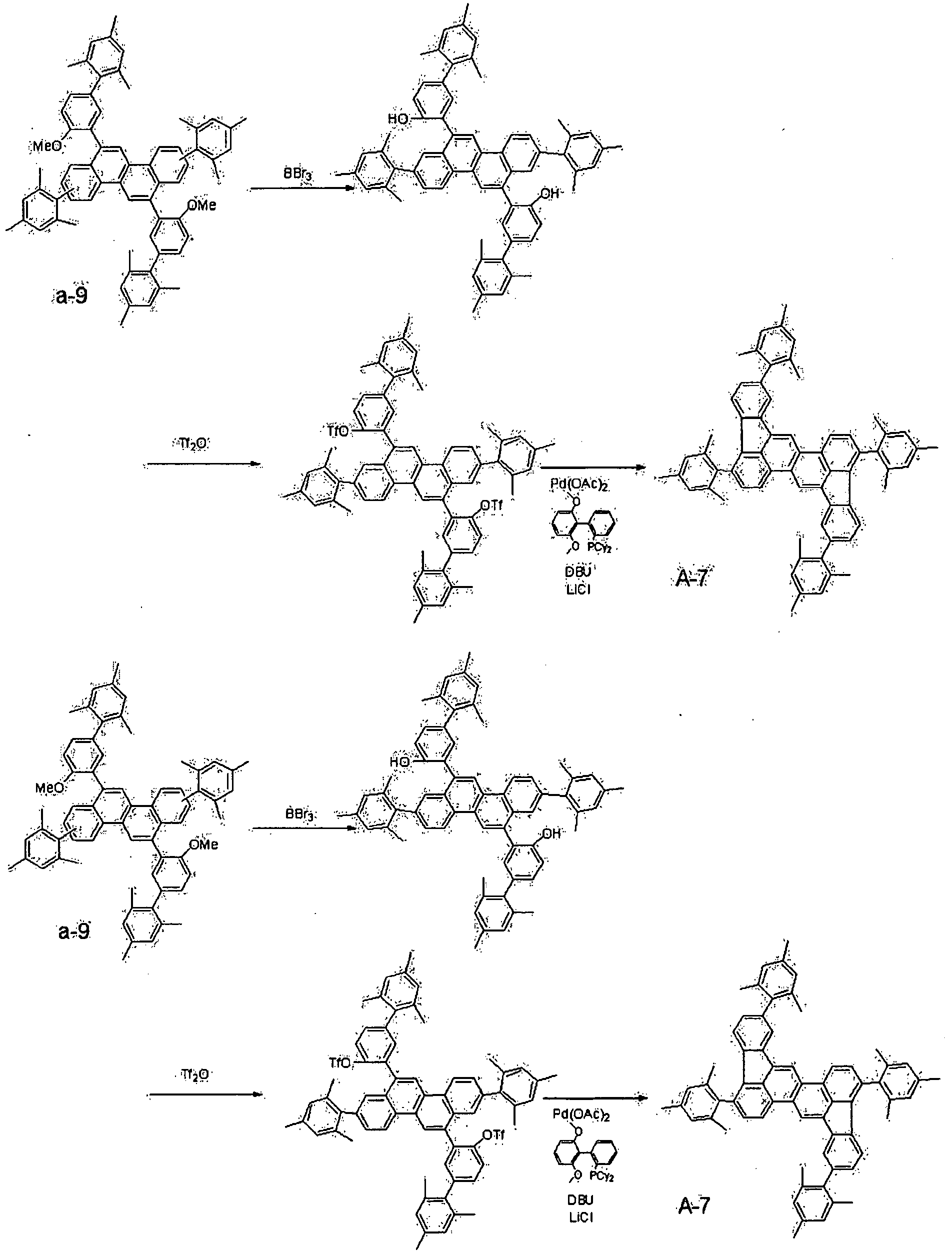

### (Synthesis Example)

Exemplified Compounds A-16, B-2, B-6, and D-1 can be synthesized in the same manner as in Example 1 except that boronic acid products and bromine products shown in Table 4 below are used instead of Compounds a-5 and a-8.

**Table 4**

| Exemplified Compound No. | Boronic acid product | Bromine product |
|---|---|---|
| A-16 | 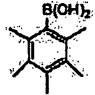 | 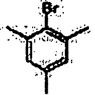 |
| B-2 | 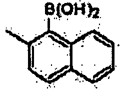 | 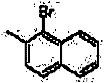 |
| B-6 | 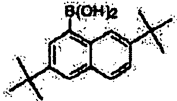 | 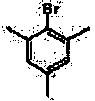 |
| D-1 | 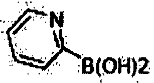 | 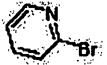 |

### (Example 3)

### (Device production)

Indium tin oxide (ITO) was formed into a film having a thickness of 120 nm to serve as an anode by a sputtering method on a glass substrate, and the resultant was used as a transparent, conductive supporting substrate. The resultant was subjected to ultrasonic cleaning with acetone and isopropyl alcohol (IPA) sequentially. Next, the resultant was subjected to boil washing with IPA, and was then dried. Further, the dried product was subjected to UV/ozone cleaning, and then the resultant was used as a transparent, conductive supporting substrate.

A solution of a compound represented by Compound b-1 below in chloroform was formed into a film having a thickness of 20 nm by a spin coating method on the transparent, conductive supporting substrate. Thus, a hole transport layer was formed.

Further, the following organic layers and electrode layers were continuously formed by vacuum deposition based on resistive heating in a vacuum chamber having a pressure of 10⁻⁵ Pa. Thus, a device was produced.
Emission layer (20 nm): Exemplified Compound A-6 (weight concentration 5%):Compound b-2 (weight concentration 95%)
Electron transport layer (40 nm): Compound b-3
Metal electrode layer 1 (0.5 nm): LiF
Metal electrode layer 2 (150 nm): Al

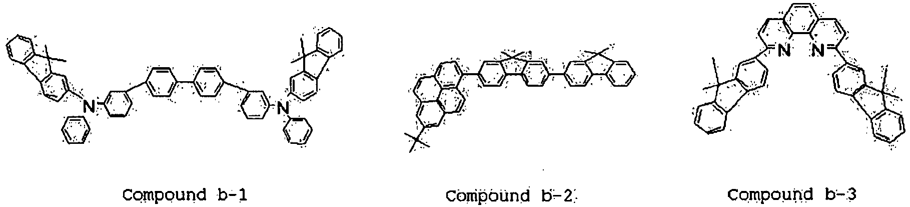

When a voltage of 4.0 V was applied to the EL device of this example, the device was observed to emit good blue light having an emission luminance of 454 cd/m² and a CIE chromaticity coodinates (0.16, 0.25).

Further, when the voltage was continuously applied for 100 hours while a current density was kept at 100 mA/cm² under a nitrogen atmosphere, the percentage by which the luminance of the device deteriorated after 100 hours as compared to the initial luminance was 20% or less, which was a small value.

### (Examples 4 to 7)

Devices were each produced in the same manner as in Example 1 except that any one of the compounds shown in Table 5 was used instead of Exemplified Compound A-6 of Example 1, and the devices were each evaluated in the same manner as in Example 1. As a result, each of the devices was observed to emit good blue light. The results are shown below.

**Table 5**

| Example | Exemplified Compound No. | Emission luminance (cd/m²) At time of application of 4.0 V | CIE chromaticity coodinates (x, y) |
|---|---|---|---|
| 4 | A-16 | 433 | (0.16, 0.26) |
| 5 | B-2 | 465 | (0.15, 0.25) |
| 6 | B-6 | 460 | (0.16, 0.26) |
| 7 | D-1 | 472 | (0.16, 0.27) |

### (Comparative Example 1) (Method of producing C-1)

C-1 can be synthesized in accordance with the following scheme.

The fluorescent spectra of C-1 in a toluene solution and in a spin-coated film, and a fluorescent spectrum in a co-deposited film of C-1 and Compound b-1 were each measured in the same manner as in Example 1. Tables 1, 2, and 3 above show the fluorescent peak wavelengths and the CIE chromaticity coodinates.

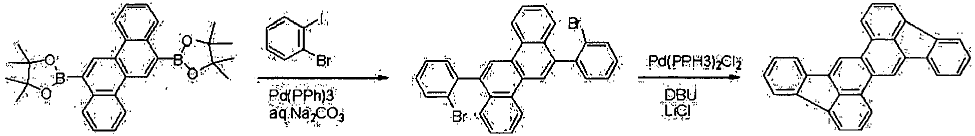

### (Comparative Example 2) (Method of producing C-2)

C-2 can be synthesized in accordance with the following scheme.

The fluorescent spectra of C-2 in a toluene solution and in a spin-coated film, and a fluorescent spectrum in a co-deposited film of C-2 and Compound b-1 were each measured in the same manner as in Example 1. Tables 1, 2, and 3 above show the fluorescent peak wavelengths and the CIE chromaticity coodinates.

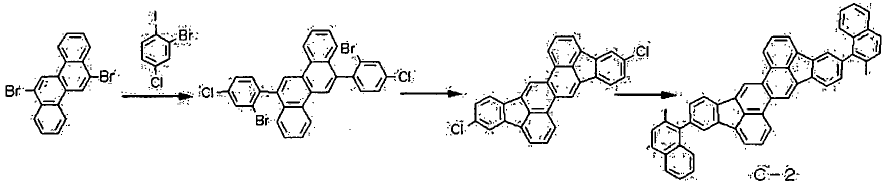

### (Comparative Example 3) (Method of producing C-3)

C-3 can be synthesized in accordance with the following scheme.

The fluorescent spectra of C-3 in a toluene solution and in a spin-coated film, and a fluorescent spectrum in a co-deposited film of C-3 and Compound b-1 were each measured in the same manner as in Example 1. Tables 1, 2, and 3 above show the fluorescent peak wavelengths and the CIE chromaticity coodinates.

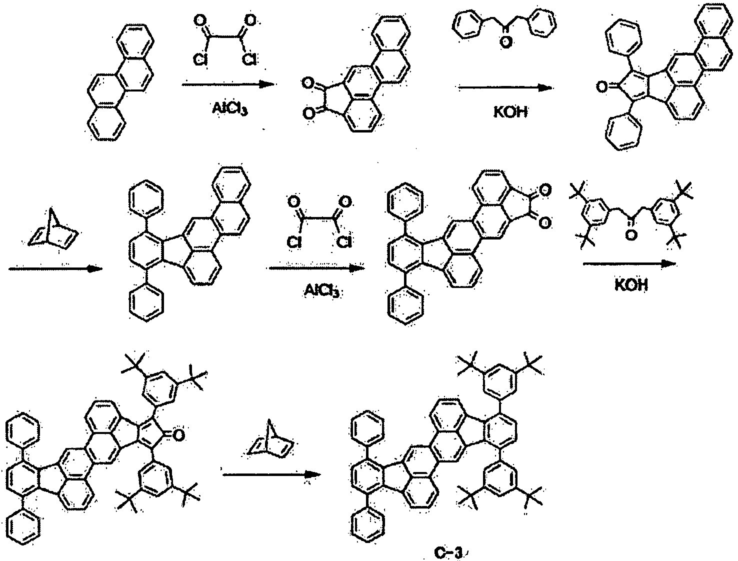

### (Comparative Example 4) (Method of producing C-4)

C-4 can be synthesized in accordance with the following scheme.

The fluorescent spectra of C-4 in a toluene solution and in a spin-coated film, and a fluorescent spectrum in a co-deposited film of C-4 and Compound b-1 were each measured in the same manner as in Example 1. Tables 1, 2, and 3 above show the fluorescent peak wavelengths and the CIE chromaticity coodinates.

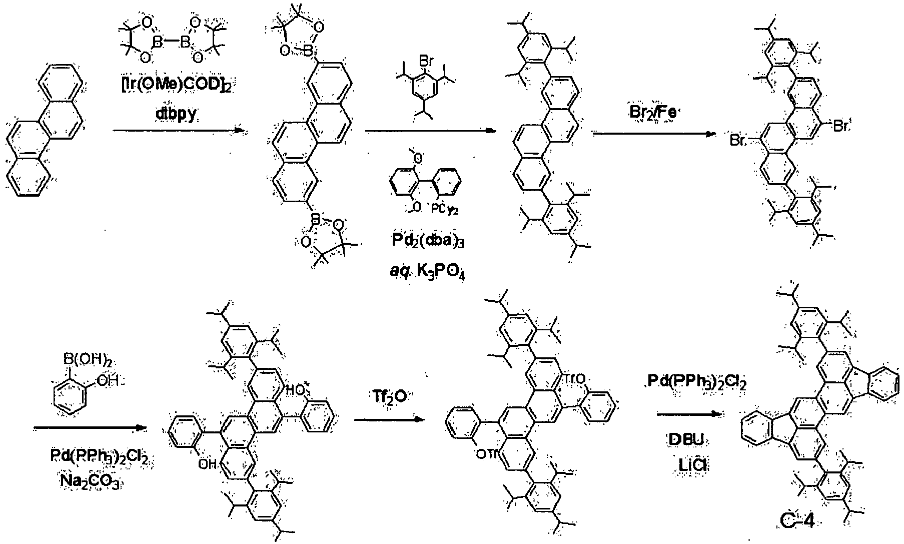

## Claims

1. A fused polycyclic compound represented by the following general formula (1): 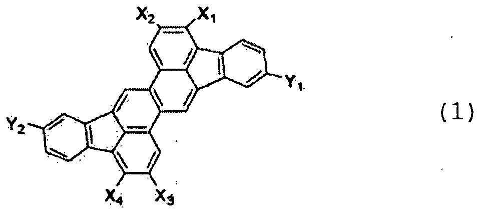 where at least one of X₁ and X₂, at least one of X₃ and X₄, and at least one of Y₁ and Y₂ are each independently selected from an aryl group and a heterocyclic group.

2. The fused polycyclic compound according to claim 1, wherein X₂, X₃, Y₁, and Y₂ are each independently selected from the aryl group and the heterocyclic group, and X₁ and X₄ each represent a hydrogen atom.

3. The fused polycyclic compound according to claim 2, wherein X₂, X₃, Y₁, and Y₂ each represent a phenyl group.

4. An organic light emitting device, comprising:
a pair of electrodes formed of an anode and a cathode; and
an organic compound layer containing an organic compound, the layer being placed between the pair of electrodes,
wherein the organic compound comprises the fused polycyclic compound according to claim 1.

5. The organic light emitting device according to claim 4, wherein the organic compound layer is an emission layer.

6. An image display apparatus, comprising:
multiple pixels; and
units for supplying electrical signals to the pixels,
wherein the pixels each have the organic light emitting device according to claim 4.

## Patentansprüche

1. Kondensierte polyzyklische Verbindung, die durch die folgende allgemeine Formel (1) dargestellt ist: 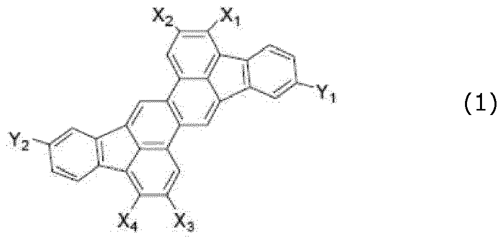 wobei zumindest eines aus X₁ und X₂, zumindest eines aus X₃ und X₄, und zumindest eines aus Y₁ und Y₂ jeweils unabhängig aus einer Arylgruppe und einer heterozyklischen Gruppe ausgewählt sind.

2. Kondensierte polyzyklische Verbindung nach Anspruch 1, wobei X₂, X₃, Y₁ und Y₂ jeweils unabhängig aus der Arylgruppe und der heterozyklischen Gruppe ausgewählt sind, und X₁ und X₄ jeweils ein Wasserstoffatom darstellen.

3. Kondensierte polyzyklische Verbindung nach Anspruch 2, wobei X₂, X₃, Y₁ und Y₂ jeweils eine Phenylgruppe darstellen.

4. Organische Licht-emittierende Vorrichtung, die umfasst:
ein Paar von Elektroden, die aus einer Anode und einer Kathode gebildet sind; und
eine organische-Verbindung-Schicht, die eine organische Verbindung enthält, wobei die Schicht zwischen dem Paar von Elektroden platziert ist,
wobei die organische Verbindung die kondensierte polyzyklische Verbindung nach Anspruch 1 umfasst.

5. Organische Licht-emittierende Vorrichtung nach Anspruch 4, wobei die organische-Verbindung-Schicht eine Emissionsschicht ist.

6. Bildanzeigevorrichtung, die umfasst:
mehrere Pixel; und
Einheiten zum Liefern elektrischer Signale zu den Pixeln,
wobei die Pixel jeweils die organische Licht-emittierende Vorrichtung nach Anspruch 4 aufweisen.

## Revendications

1. Composé polycyclique condensé représenté par la formule générale(1) : 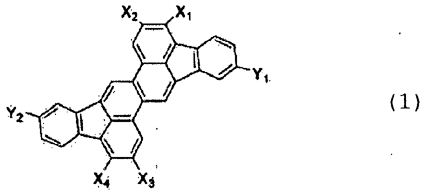 où au moins un de X₁ et X₂, au moins un de X₃ et X₄ et au moins un de Y₁ et Y₂ sont chacun choisis indépendamment parmi un groupe aryle et un groupe hétérocyclique.

2. Composé polycyclique condensé selon la revendication 1, dans lequel X₂, X₃, Y₁ et Y₂ sont chacun choisis indépendamment parmi le groupe aryle et le groupe hétérocyclique, et X₁ et X₄ représentent chacun un atome d'hydrogène.

3. Composé polyclyclique condensé selon la revendication 2, dans lequel X₂, X₃, Y₁ et Y₂ représentent chacun un groupe phényle.

4. Dispositif électroluminescent organique, comprenant :
une paire d'électrodes formée d'une anode et d'une cathode ; et
une couche de composé organique contenant un composé organique, la couche étant placée entre la paire d'électrodes,
dans laquelle le composé organique comprend le composé polycyclique condensé selon la revendication 1.

5. Dispositif électroluminescent organique selon la revendication 4, dans lequel la couche de composé organique est une couche d'émission.

6. Appareil d'affichage d'image, comprenant :
de multiples pixels ; et
des unités pour fournir des signaux électriques aux pixels,
dans lequel les pixels ont chacun le dispositif émetteur de lumière organique selon la revendication 4.
